# EUROPÄISCHE PATENTSCHRIFT

(11) **EP 1 434 761 B1**
(45) Veröffentlichungstag und Bekanntmachung des Hinweises auf die Patenterteilung: **24.02.2010**
(21) Anmeldenummer: 02785190.6
(22) Anmeldetag: 10.10.2002
(51) Int. Cl.: C07C 281/18, A61K 31/155, A61P 35/00

(54) **SALZE VON GUANIDINDERIVATEN UND PHARMAZEUTISCHE ZUBEREITUNGEN DARAUS**
SALTS OF GUANIDINE DERIVATIVES AND PHARMACEUTICAL PREPARATIONS CONSISTING THEREOF
SELS DE DERIVES DE LA GUANIDINE ET PREPARATIONS PHARMACEUTIQUES ASSOCIEES

(30) Priorität: 10.10.2001 DE 10149919
(43) Veröffentlichungstag der Anmeldung: 07.07.2004
(73) Patentinhaber: Biosphings Aktiengesellschaft, 60329 Frankfurt am Main (DE)
(72) Erfinder: AMTMANN, Eberhard, 69121 Heidelberg (DE)
(74) Vertreter: Wagner, Jutta
(86) Internationale Anmeldenummer: PCT/EP2002/011338
(87) Internationale Veröffentlichungsnummer: WO 2003/031396

(56) Entgegenhaltungen:
- WO-A-97/45401
- DE-A1- 2 459 051
- GB-A- 1 040 049
- DATABASE WPI Section Ch, Week 198310 Derwent Publications Ltd., London, GB; Class B03, AN 1983-23831K XP002229699 & JP 58 015913 A (MITSUBISHI CHEM IND LTD) , 29. Januar 1983 (1983-01-29)
- TETRAHEDRON LETTERS., Bd. 40, 1999, Seiten 17-20, NLELSEVIER, AMSTERDAM.
- TETRAHEDRON LETTERS., Bd. 40, 1999, Seiten 7067-7071, NLELSEVIER, AMSTERDAM.
- JOURNAL OF HETEROCYCLIC CHEMISTRY., Bd. 12, 1975, Seiten 1143-1153, USHETERO CORP., TAMPA, FL.
- JOURNAL OF THE CHEMICAL SOCIETY., 1948, Seiten 1939-1945, GBCHEMICAL SOCIETY. LETCHWORTH.

## Beschreibung

Die Erfindung betrifft pharmazeutische Zubereitungen, die neue Salze von Guanidinderivaten als Wirkstoff enthalten, sowie deren Herstellung.

Die WO 97/45401 offenbart Guanidinderivate der allgemeinen Formel (1) 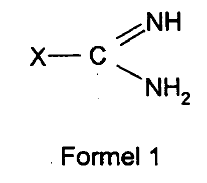 bei denen X für die Gruppe -R¹, -NHR¹, -NH-NH-CHR¹R² oder NH-N=CR¹R² steht, wobei R¹ und R² unabhängig voneinander für Wasserstoff, ein lineares oder verzweigtes C₃-C₂₀-Alkyl- oder C₃-C₂₀-Cycloalkyl-, Adamantyl-, Norbornyl-, Tricyclodecyl- oder Benzyl-, Pyridyl-, Indolyl-, Chinolyl-, Anthracyl-, Phenantryl-, Perinaphtyl- oder Chinuclidinyl-Radikal stehen. Die Einsatz dieser Verbindungen als Wirkstoff in pharmazeutischen Zubereitungen beruht insbesondere auf der Hemmung von Sphingomyelinasen. Als Beispiele für Verbindungen entsprechend Formel 1 werden ausschließlich die freien Basen beschrieben.

Auf Grund ihrer Detergensstruktur weisen die beschriebenen Basen und ihre durch Neutralisation mit anorganischen Säuren oder durch Lösen in gepufferter physiologischer Kochsalzlösung erhaltenen Salze starke hämolytische und gewebsschädigende Eigenschaften auf. Weiterhin bilden sich aus anorganischen Salzen in Anwesenheit von anorganischen Phosphationen in wässrigen Lösungen unlösliche Phosphate der Guanidine. Deshalb werden sie im Blut bei relativ niedrigen Konzentrationen ausgefällt und damit pharmazeutisch unwirksam gemacht. Aus diesen Gründen ist die pharmazeutische Verwendbarkeit von Guanidinderivaten stark eingeschränkt.

Es stellte sich daher die Aufgabe, eine physiologisch verträgliche und gleichzeitig wirksame Darreichungsform für die Guanidinderivate bereitzustellen.

Es wurde nun überraschend gefunden, dass Salze, die aus der Umsetzung aktiver Guanidinderivate mit bestimmten organischen Säuren hervorgehen, eine um bis zum hundertfachen geringere hämolytische Wirkung und eine hervorragende lokale Verträglichkeit, bei gleichbleibender Wirksamkeit, d.h. insbesondere Sphingomyelinasehemmung, besitzen. Weiterhin werden sie durch anorganische Phosphate nicht ausgefällt. Aus diesem Grunde besitzen die erfindungsgemäßen Salze eine stark erhöhte Dosierbarkeit.

Die oben genannte Aufgabe wird daher gelöst durch pharmazeutische Zubereitungen enthaltend Salze der allgemeinen Formel (2): 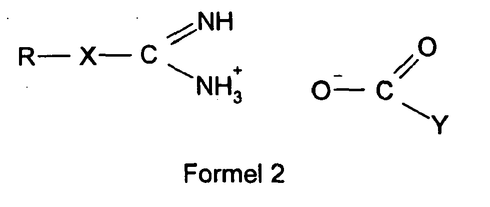
wobei X für -CH₂-NH-NH- oder für -CH=N-NH- und R für einen geraden oder verzweigten C₁-C₃₀-Alkyl-, C₃-C₂₀-Cycloalkyl-, oder Tricyclodecyl- Rest steht, der durch eine oder mehrere Hydroxylgruppen, C₁-C₄-Alkoxygruppen, C₁-C₄-Alkylgruppen und/oder ein oder mehrere Halogenatome oder eine oder mehrere Aminogruppen substituiert sein kann, und
Y für einen geraden oder verzweigten C₁-C₁₂-Alkyl-, C₃-C₈-Cycloalkyl-, Benzyl-, Furyl- oder Pyridyl-Rest steht, der durch eine oder mehrere Hydroxylgruppen, Carbonsäuregruppen, C₁-C₄-Alkoxygruppen., C₁-C₄-Alkylgruppen und/oder ein oder mehrere Halogenatome oder eine oder mehrere Aminogruppen substituiert sein kann, oder ansteht der Carbonsäuren die entsprechend substituierten Sulfonsäuren oder Phosphonsäuren eingesetzt werden.

Bevorzugt steht R für Pentyl-, Hexyl-, Heptyl-, Octyl-, Nonyl-, Decyl-, Undecyl-, Dodecyl-, Tridecyl-, Cyclododecyl-, Tricyclo[5,2,1,0^{2,6}]-decyl-, Bicyclo[2,2,1]-Cyclohexyl oder Toluyl. Besonders vorteilhaft ist ein Decylrest.

X steht vorzugsweise für -CH=N-NH-.

Besonders vorteilhaft für Y sind Methyl, Ethyl, Propyl, iso-Propyl, Butyl, Pentyl, Hexyl, Heptyl, Octyl, Nonyl, Decyl, Undecyl und Dodecyl sowie Hydroxyethyl- und 2-Hydroxy-2,3-Dicarbonsäurepropyl. Insbesondere bevorzugt sind Methyl, Ethyl, Octyl, Nonyl, Decyl, Undecyl und Dodecyl sowie Hydroxyethyl- und 2-Hydroxy-2,3-Dicarbonsäurepropyl.

Besonders bevorzugte erfindungsgemäße Verbindungen sind Undecylaminoguanidinacetat, Undecylaminoguanidinlactat, Undecylaminoguanidinönanthat, Undecylaminoguanidinpelargonat und insbesondere Undecylidenaminoguanidinacetat, Undecylidenaminoguanidinlactat, Undecylidenaminoguanidinönanthat und Undecylidenaminoguanidinpelargonat.

Anstelle der Carbonsäuren können auch die entsprechend substituierten Sulfonsäuren oder Phosphonsäuren eingesetzt werden. Selbstverständlich bezieht sich die Erfindung gegebenenfalls sowohl auf die reinen optischen Isomeren als auch auf deren Mischungen sowie Racemate, Tautomere oder Stereoisomere.

Die erfindungsgemäßen Salze der Guanidinderivate weisen keine Detergenswirkung mehr auf, da die polare Gruppe der Guanidine mit einem hydrophoben Säureanion verbunden ist. Dadurch sind diese Verbindungen physiologisch gut verträglich. Überraschenderweise sind die erfindungsgemäßen Guanidinsalze bis zu Konzentrationen von 1000 mmol/l gut wasserlöslich und werden durch Phosphationen nicht aus der wässrigen Lösung ausgefällt. Die Wirksamkeit, insbesondere die Sphingomyelinasehemmung, wird dagegen nicht oder nur unerheblich beeinträchtigt.

Die Herstellung der Guanidine ist z.B. aus der WO 97/45401 bekannt. Die Herstellung der erfindungsgemäßen Guanidinsalze kann in einfacher Weise durch Zusammenbringen der freien Basen mit den entsprechenden freien Säuren erfolgen. Dies ist sowohl in geeigneten Lösungsmitteln, vor allem in Wasser, als auch in Substanz, insbesondere wenn die Säure und/oder die Base flüssig ist, möglich. Es kann vorteilhaft sein, die Säure im Überschuss zuzufügen.

Die Erfindung betrifft weiter pharmazeutische Zubereitungen, die zur Behandlung von Tumorerkrankungen, Autoimmunerkrankungen, Herz-Kreislauferkrankungen, Infektionen und insbesondere Viruserkrankungen eingesetzt werden können. Die Wirkstoffe eignen sich auch zur Prophylaxe. Bevorzugte Wirkstoffe sind: Undecylidenaminoguanidinacetat, Undecylidenaminoguanidinlactat und insbesondere Undecylidenaminoguanidinönanthat und Undecylidenaminoguanidinpelargonat.

Die erfindungsgemäßen Zubereitungen enthalten üblicherweise neben dem erfindungsgemäßen Wirkstoff Hilfs- und Zusatzstoffe, die die Konfektionierung zu Darreichungsformen ermöglichen. Es können auch z.B. für Kombinationspräparate weitere Wirkstoffe zugefügt sein, sofern sie chemisch mit den erfindungsgemäßen Wirkstoffen kompatibel sind. Als Darreichungsformen kommen in Betracht: flüssige Zubereitungen zur Injektion; Tabletten, Kapseln, Pulver, Lösungen, Suspensionen oder Elixiere zur oralen Applikation; Salben, Cremes, Emulsionen oder Lotionen zur topischen Anwendung; Pulver und Lösungen zur Inhalation und Suppositorien

Die Verfahren zur Herstellung von Darreichungsformen sind dem Fachmann bekannt. Neben der Verarbeitung der erfindungsgemäßen Salze zu den Darreichungsformen können diese Wirkstoffe auch durch Bereitstellen der freien Base und Säure, vorzugsweise in equimolaren Mengen, und Verarbeitung dieser Mischung gegebenenfalls mit den weiteren Hilfs- und Zusatzstoffen zu Darreichungsformen verarbeitet werden. Dabei wird das Salz in situ bzw. bei der Freisetzung aus der Darreichungsform im Körper erzeugt. Brauchbare Konzentrationen liegen im Bereich von 0,5 bis 30 % Wirkstoff in Injektionslösungen und bei mindestens 1 % Wirkstoff in Zubereitungen für die restlichen Darreichungsformen. Die tägliche Dosis beträgt bei Erwachsenen zwischen 5 und 1000 mg

Die folgenden Beispiele sollen die Erfindung näher veranschaulichen, ohne sie jedoch darauf zu beschränken.

### Beispiel 1: Herstellung von Undecylidenaminoguanidinacetat

23,4g (0,1 Mol) Undecylidenaminoguanidin (d.h C₁₀H₂₁-CH=N-NH-C(NH)-NH₂, im folgenden C11AG abgekürzt) werden in 234 ml destilliertem Wasser aufgeschlämmt und nach Erwärmung auf 60 °C unter Rühren langsam mit 6,005 g Eisessig versetzt. Nach 30 min Rühren wird abfiltriert und auf 0°C abgekühlt. Das auskristallisierte Salz wird abfiltriert und mit eiskaltem Wasser gewaschen. Es werden 27,8 g des Acetates erhalten (Ausbeute 94,5 %).

### Beispiel 2: Herstellung von Undecylidenaminoguanidinönanthat

23,4g (0,1 Mol) Undecylidenaminoguanidin (C11AG) werden in 234 ml destilliertem Wasser aufgeschlämmt und nach Erwärmung auf 60 °C unter Rühren langsam mit 13,02 g Önanthsäure versetzt. Nach 30 min Rühren wird abfiltriert und auf 0°C abgekühlt. Das auskristallisierte Salz wird abfiltriert und mit eiskaltem Wasser gewaschen. Es werden 22 g des Önanthats erhalten (Ausbeute 60,4 %).

### Beispiel 3: Herstellung von Undecylidenaminoguanidinpelargonat

23,4g (0,1 Mol) Undecylidenaminoguanidin (C11AG) werden in 234 ml destilliertem Wasser aufgeschlämmt und nach Erwärmung auf 60 °C unter Rühren langsam mit 15,82 g Pelargonsäure versetzt. Nach 30 min Rühren wird abfiltriert und auf 0°C abgekühlt. Das auskristallisierte Salz wird abfiltriert und mit eiskaltem Wasser gewaschen. Es werden 36,1 g des Pelargonats erhalten (Ausbeute 92 %).

### Beispiel 4: Herstellung von weiteren Undecylidenaminoguanidinsalzen

1,1 mMol Säure und 1mMol C11AG (234 mg) wurden in 3 ml Ethylacetat gelöst. Überschüssige Säure wurde durch dreimaliges Ausschütteln mit destilliertem Wasser entfernt. Nach dem Trocknen im Vakuum wurde das Salz in einer Ausbeute von mehr als 90 % erhalten. In Tabelle 1 sind die Löslichkeiten der erhaltenen Salze und ihreSchmelzpunkte, sowie die entsprechenden Daten der Salze aus den Beispielen 1 - 3 zusammengestellt.

**Tabelle 1**

| Säureanion | Löslichkeit in Wasser | Schmelzpunkt |
|---|---|---|
| Acetat | > 10 mg/ml | 90 °C |
| Lactat | > 10 mg/ml | 16 °C |
| Isobutyrat | > 10 mg/ml | < 4 °C |
| Heptanat | > 10 mg/ml | 89 °C |
| Nonat | > 10 mg/ml | 82 °C |
| Decanat | 1 mg/ml | 48 °C |
| Undecanat | 0,1 mg/ml | 81°C |
| Dodecanat | 0,01 mg/ml | 68 °C |
| Dodecylphosphonat | 0,1 mg/ml | 78 °C |
| Undecylenat | 0,1 mg/ml | 62 °C |
| Chlorid | > 10 mg/ml | 63 °C |
| Palmitat | 0,01 mg/l | 72 °C |
| Stearat | 0,01 mg/ml | 81 °C |

Es zeigt sich, dass auch recht langkettige Säuren noch gut wasserlösliche Salze ergeben. Die weniger wasserlöslichen Salze können aber in Einzelfällen für spezielle Anwendungen geeigneter sein.

### Beispiel 5: hämolytische Aktivität verschiedener Salze von C11AG

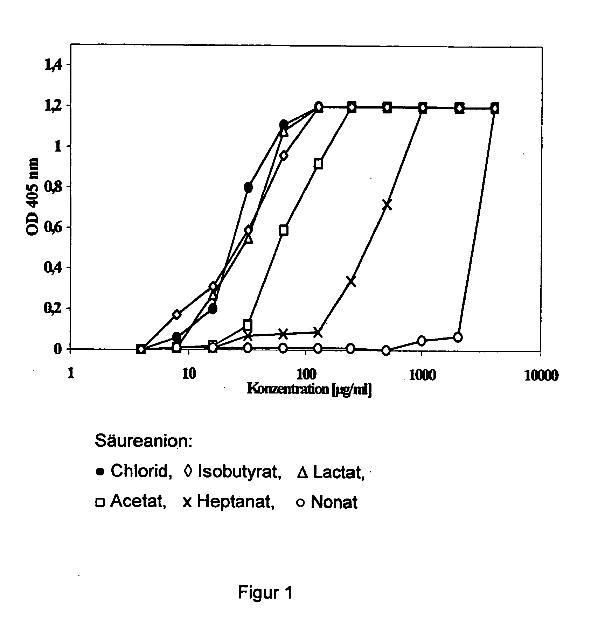

Die Salze der Milchsäure, Essigsäure, Salzsäure, Önanthsäure und Pelargonsäure wurden wie oben beschrieben hergestellt. Mäuseblut (aus der Schwanzvene entnommen) wurde 1:100 in isotonischer Glukoselösung verdünnt. Zu je 0,1 ml verdünntem Mäuseblut wurden die verschiedenen C11AG-Salze in Endkonzentrationen von 4-4000 µg/ml zugegeben. Nach Mischen und Inkubation bei Raumtemperatur für 20 min wurde kurz zentrifugiert und die optische Dichte bei 405 nm vermessen. Die optische Dichte ist ein Maß für die Menge des aus den Erythrozyten freigesetzten Hämoglobins. Figur 1 zeigt die gemessene optische Dichte in Abhängigkeit von der logarithmisch dargestellten Konzentration des Guanidinderivates. Die Endkonzentration verdoppelt sich jeweils von einem Messpunkt zum nächsten. Man erkennt, dass die erfindungsgemäßen Guanidinderivate erst bei Konzentrationen von 32 µg/ml und darüber eine nennenswerte hämolytische Aktivität zeigen, während Chlorid bereits zwischen 8 und 16 µg/ml deutlich hämolytisch wirkt.

### Beispiel 6: Löslichkeit von C11AG-Salzen in Phosphatpuffer

Es wurde eine 1 M Kaliumphosphat-Pufferlösung, pH 7,4, hergestellt. Eine Verdünnungsreihe von 1000 mM bis 1 mM Phosphat wurde mit den C11AG-Salzen der Milchsäure, Essigsäure, Salzsäure, Önanthsäure und Pelargonsäure versetzt, sodass sich jeweils eine Endkonzentration des C11AG-Salzes von 4 mM ergab. Die Bildung von Kristallen wurde mikroskopisch bei 40 facher Vergrößerung kontrolliert. Die Ergebnisse sind in Tabelle 2 angegeben. Während das Chlorid bereits bei 1mM unlösliche Phosphate bildet, fallen bei Acetat und Lactat erst bei 10 mM Phosphate aus und bei Önanthat und Pelargonat sind selbst Lösungen mit 1000 mM Phosphationen stabil.

**Tabelle 2**

| Salz | Konzentration | Kristalle |
|---|---|---|
| Chlorid | 1 mM-1000mM | ja |
| Acetat | 1 mM | nein |
| Acetat | 10 mM-1000 mM | ja |
| Lactat | 1 mM | nein |
| Lactat | 10 mM-1000 mM | ja |
| Önanthat | 1 mM - 1000 mM | nein |
| Pelargonat | 1 mM-1000 mM | nein |

### Beispiel 7: Hemmung der neutralen Sphingomyelinase

Die Salze der Milchsäure, Essigsäure, Salzsäure, Önanthsäure und Pelargonsäure wurden wie oben beschrieben oder in analoger Weise hergestellt. Paranitrophenylphosphorylcholin wurde in 0,1 M Tris bei pH 7,2 und 10 mM MgCl₂ zu 1 mg/ml gelöst. Nach Zugabe von 0,1 Einheit neutraler Sphingomyelinase aus Bazillus cereus wurden die verschiedenen Salze von C11AG in Konzentrationen zwischen 0,1 und 1000 µg/ml zugegeben. Nach Inkubation bei 37°C für 24 h wurde die optische Dichte bei 405 nm gemessen. Die optische Dichte ist ein Maß für die Menge an gespaltenem Substrat. Aus Dosis-Wirkungskurven wurde die Konzentration, bei der eine Hemmung der Enzymaktivität um 50 % (IC50) eintritt, entnommen. Die Ergebnisse sind in Tabelle 3 aufgelistet. Es zeigt sich, dass die Wirksamkeit teilweise sogar verbessert wird, jedenfalls aber in keinem Fall wesentlich unter diejenige des Chlorids sinkt.

**Tabelle 3**

| **Salz** | IC50 [µg/ml] |
|---|---|
| Chlorid | 0,8 |
| Acetat | 0,85 |
| Lactat | 0,9 |
| Önanthat | 0,75 |
| Pelargonat | 0,95 |

### Beispiel 8: physiologische Verträglichkeit verschiedener C11AG-Salze

Aus den Salzen der Essigsäure, Salzsäure, Önanthsäure und Pelargonsäure wurden Lösungen in isotonischer Glukoselösung in Konzentrationen von 4 mM, 20 mM, 40 mM und 120 mM hergestellt. Jeweils 0,1 ml wurde 3 haarlosen Mäusen (Stamm Swiss, Nu/Nu) subkutan appliziert. Nach 24 h Stunden wurde der Grad der Gewebsschädigung protokolliert. Die Ergebnisse zeigt Tabelle 4, es bedeuten keine sichtbaren Schädigungen = -, Rötung= +, Ödeme = ++ und Nekrosen = +++ Man erkennt, dass das Chlorid bereits bei 20 mM zu Rötungen und Ödemen führt. Das Acetat ist bis 20 mM gut verträglich und bewirkt erst bei 120mM die Schädigung, die beim Chlorid schon bei 20 mM auftreten. Die bevorzugten Önanthate und Pelargonate zeigen selbst bei 120 mM keinerlei Schädigung.

**Tabelle 4**

| Salz | Konzentration | Befund |
|---|---|---|
| Chlorid | 4 mM | 1 x +, 2 x - |
| Chlorid | 20 mM | 1 x +, 2 x ++ |
| Chlorid | 40 mM | 2 x ++, 1 x +++ |
| Chlorid | 120 mM | 3 x +++ |
| Acetat | 4 mM - 20 mM | 3 x - |
| Acetat | 120 mM | 1 x +, 2 x ++ |
| Önanthat | 4 mM - 120 mM | 3 x - |
| Pelargonat | 4 mM-120 mM | 3 x - |

## Patentansprüche

1. Pharmazeutische Zubereitungen enthaltend ein oder mehrere Salze von Guanidinderivaten entsprechend der Formel 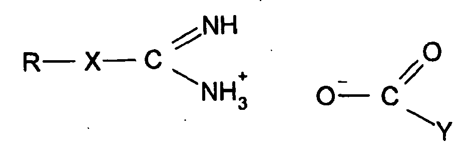 wobei
X für -CH₂-NH-NH- oder für -CH=N-NH- und R für einen geraden oder verzweigten C₁-C₃₀-Alkyl-, C₃-C₂₀-Cycloalkyl- oder Tricyclodecyl-Rest steht, der durch eine oder mehrere Hydroxylgruppen, C₁-C₄-Alkoxygruppen, C₁-C₄-Alkylgruppen und/oder ein oder mehrere Halogenatome oder eine oder mehrere Aminogruppen substituiert sein kann, und
Y für einen geraden oder verzweigten C₁-C₁₂-Alkyl-, C₃-C₈-Cycloalkyl-, Benzyl-, Furyl- oder Pyridyl-Rest steht, der durch eine oder mehrere Hydroxylgruppen, Carbonsäuregruppen, C₁-C₄-Alkoxygruppen, C₁-C₄-Alkylgruppen und/oder ein oder mehrere Halogenatome oder eine oder mehrere Aminogruppen substituiert sein kann, oder statt der Carbonsäuregruppe O-CO-Y die entsprechend substituierte Sulfon- oder Phosphonsäuregruppe vorhanden ist.

2. Zubereitung gemäß Anspruch 1, **dadurch gekennzeichnet, dass** R für einen Pentyl-, Hexyl-, Heptyl-, Octyl-, Nonyl-, Decyl-, Undecyl-, Dodecyl-, Tridecyl-, Cyclododecyl-, Tricyclo[5,2,1,0^{2,6}]-decyl- oder Norbornyl- und insbesondere für einen Decylrest steht.

3. Zubereitung gemäß Anspruch 1 oder 2, **dadurch gekennzeichnet, dass** eine Carbonsäuregruppe O-CO-Y vorhanden ist

4. Zubereitung gemäß Anspruch 1, 2 oder 3, **dadurch gekennzeichnet, dass** Y Methyl, Ethyl, Propyl, iso-Propyl, Butyl, Pentyl, Hexyl, Heptyl, Octyl, Nonyl, Decyl, Undecyl, Dodecyl, Hydroxyethyl- oder 2-Hydroxy-2,3-Dicarbonsäurepropyl ist.

5. Zubereitung gemäß Anspruch 3, **dadurch gekennzeichnet, dass** X für - CH=N-NH- steht.

6. Zubereitung gemäß Anspruch 1, **dadurch gekennzeichnet, dass** das Salz Undecylidenaminoguanidinacetat, Undecylidenaminoguanidinlactat und insbesondere Undecylidenaminoguanidinönanthat oder Undecylidenaminoguanidinpelargonat ist.

7. Zubereitung gemäß einem der Ansprüche 1 bis 6, **dadurch gekennzeichnet, dass** übliche Zusatz- und Hilfsstoffe enthalten sind.

8. Verfahren zur Herstellung einer pharmazeutischen Zubereitung gemäß einem der Ansprüche 1 bis 7, **dadurch gekennzeichnet, dass** ein Salz oder mehrere Salze von Guanidinderivaten mit üblichen Zusatz- bzw. Hilfsstoffen zu einer Darreichungsform verarbeitet wird/werden.

9. Verfahren gemäß Anspruch 8, **dadurch gekennzeichnet, dass** ein Salz durch Bereitstellen von etwa equimolaren Mengen der entsprechenden Base und Säure und Verarbeitung mit üblichen Zusatz- bzw. Hilfsstoffen zu einer Darreichungsform verarbeitet wird.

10. Verwendung einer pharmazeutischen Zubereitung gemäß einem der Ansprüche 1 bis 7 zur Herstellung von Arzneimitteln zur Behandlung von Tumorerkrankungen, Autoimmunerkrankungen, Herz-Kreislauferkrankungen, Infektionen oder-Viruserkrankungen.

## Claims

1. Pharmaceutical preparations containing one or more salts of guanidine derivatives corresponding to the formula 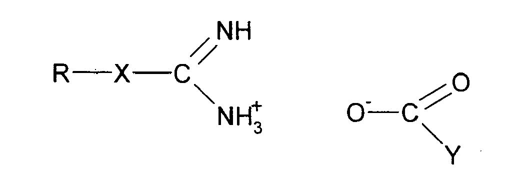
wherein X represents -CH₂-NH-NH- or -CH=N-NH-, and R represents a linear or branched C₁-C₃₀ alkyl, C₃-C₂₀ cycloalkyl, or tricyclodecyl residue, which can be substituted by one or more hydroxyl groups, C₁-C₄ alkoxy groups, C₁-C₄ alkyl groups and/or one or more halogen atoms or one or more amino groups, and
Y represents a linear or branched C₁-C₁₂ alkyl, C₃-C₈ cycloalkyl, benzyl, furyl or pyridyl residue, which can be substituted by one or more hydroxyl groups, carboxylic acid groups, C₁-C₄ alkoxy groups, C₁-C₄ alkyl groups and/or one or more halogen atoms or one or more amino groups or that a sulfonic or phosphonic acid group is present in place of the carboxylic acid group, O-CO-Y.

2. Preparation according to Claim 1, **characterized in that** R represents a pentyl, hexyl, heptyl, octyl, nonyl, decyl, undecyl, dodecyl, tridecyl, cyclododecyl, tricyclo[5,2,1,0^{2,6}]-decyl or norbornyl residue and, in particular, a decyl residue.

3. Preparation according to claim 1 or 2, **characterized in that** a carbonic acid group O-CO-Y is present.

4. Preparation according to Claim 1, 2 or 3, **characterized in that** Y is methyl, ethyl, propyl, isopropyl, butyl, pentyl, hexyl, heptyl, octyl, nonyl, decyl, undecyl, dodecyl, hydroxyethyl or 2-hydroxy-2,3-dicarboxylic acid propyl.

5. Preparation according to Claim 3, **characterized in that** X represents -CH=N-NH-.

6. Preparation according to Claim 1, **characterized in that** the salt is undecylideneaminoguanidine acetate, undecylideneaminoguanidine lactate, and, in particular, undecylideneaminoguanidine oenanthate and undecylideneaminoguanidine pelargonate.

7. Preparation according to anyone of the Claims 1 to 6, **characterized in that** common additives and excipients are contained therein.

8. Method for the preparation of a pharmaceutical preparation according to anyone of Claims 1-7, **characterized in that** a salt of a guanidine derivative is processed with common additives and/or excipients to a form of administration.

9. Method according to Claim 8, **characterized in that** a salt is processed to a form of administration by providing approx. equimolar amounts of the corresponding base and acid and processing with common additives and/or excipients.

10. Use of a pharmaceutical preparation according to anyone of Claims 1-7 for manufacturing of medicaments for the treatment of tumor diseases, autoimmune diseases, cardiovascular diseases, infections or viral diseases.

## Revendications

1. Compositions pharmaceutiques contenant un ou plusieurs sels de dérivés de guanidine, répondant à la formule 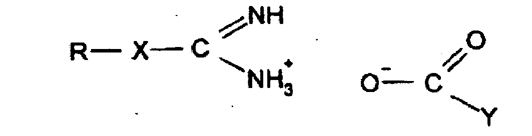 dans laquelle
X représente un groupe -CH₂-NH-NH- ou représente un groupe -CH=N-NH- et R représente un radical alkyle en C₁-C₃₀ à chaîne droite ou ramifiée, un radical cycloalkyle en C₃-C₂₀ ou un radical tricyclodécyle, qui peut être substitué par un ou plusieurs groupes hydroxyle, un ou plusieurs groupes alcoxy en C₁-C₄, un ou plusieurs groupes alkyle en C₁-C₄ et/ou un ou plusieurs atomes d'halogène ou un ou plusieurs groupes amino, et
Y représente un radical alkyle en C₁-C₁₂ à chaîne droite ou ramifiée, un radical cycloalkyle en C₃-C₈, un radical benzyle, un radical furyle ou un radical pyridyle qui peut être substitué par un ou plusieurs groupes hydroxyle, un ou plusieurs groupes d'acides carboxyliques, un ou plusieurs groupes alcoxy en C₁-C₄, un ou plusieurs groupes alkyle en C₁-C₄ et/ou un ou plusieurs atomes d'halogène ou un ou plusieurs groupes amino, ou bien un groupe d'acide sulfonique ou d'acide phosphonique substitué de manière correspondante est présent à la place du groupe d'acide carboxylique O-CO-Y.

2. Préparation selon la revendication 1, **caractérisée en ce que** R représente un radical pentyle, un radical hexyle, un radical heptyle, un radical octyle, un radical nonyle, un radical décyle, un radical undécyle, un radical dodécyle, un radical tridécyle, un radical cyclododécyle, un radical tricyclo[5.2.1.0^{2.6}]décyle ou un radical norbornyle, et en particulier un radical décyle.

3. Préparation selon la revendication 1 ou 2, **caractérisée en ce qu'**un groupe d'acide carboxylique O-CO-Y est présent.

4. Préparation selon la revendication 1, 2 ou 3, **caractérisée en ce que** Y représente un groupe méthyle, un groupe éthyle, un groupe propyle, un groupe isopropyle, un groupe butyle, un groupe pentyle, un groupe hexyle, un groupe heptyle, un groupe octyle, un groupe nonyle, un groupe décyle, un groupe undécyle, un groupe dodécyle, un groupe hydroxyéthyle ou un groupe 2-hydroxy-2,3-acide dicarboxylique-propyle.

5. Préparation selon la revendication 3, **caractérisée en ce que** X représente un groupe -CH=N-NH-.

6. Préparation selon la revendication 1, **caractérisée en ce que** le sel est l'acétate de undécylidène-aminoguanidine, le lactate de undécylidène-aminoguanidine et en particulier l'oenanthate de undécylidène-aminoguanidine ou le pelargonate de undécylidène-aminoguanidine.

7. Préparation selon l'une quelconque des revendications 1 à 6, **caractérisée en ce qu'**elle contient des additifs et des adjuvants habituels.

8. Procédé pour la formulation d'une préparation pharmaceutique selon l'une quelconque des revendications 1 à 7, **caractérisé en ce qu'**on traite un sel ou plusieurs sels de dérivés de guanidine avec des additifs respectivement des adjuvants habituels pour obtenir une forme d'administration.

9. Procédé selon la revendication 8, **caractérisé en ce qu'**on traite un sel via la préparation de quantités approximativement équimolaires de la base correspondante et de l'acide correspondant et par traitement avec des additifs respectivement des adjuvants habituels pour obtenir une forme d'administration.

10. Utilisation d'une préparation pharmaceutique selon l'une quelconque des revendications 1 à 7, pour la préparation de médicament destiné au traitement de maladies tumorales, de maladies auto-immunes, de maladies cardio-vasculaires, d'infections ou de maladies virales.
